# EUROPÄISCHE PATENTSCHRIFT

(11) **EP 2 923 720 B1**
(45) Veröffentlichungstag und Bekanntmachung des Hinweises auf die Patenterteilung: **15.08.2018**
(21) Anmeldenummer: 15000827.4
(22) Anmeldetag: 20.03.2015
(51) Int. Cl.: A61M 16/00

(54) **VORRICHTUNG ZUR THERAPIE PERIODISCHER ATMUNG**
DEVICE FOR PERIODIC BREATHING THERAPY
DISPOSITIF DE THÉRAPIE DE LA RESPIRATION PÉRIODIQUE

(30) Priorität: 28.03.2014 DE 102014004447
(43) Veröffentlichungstag der Anmeldung: 30.09.2015
(73) Patentinhaber: Löwenstein Medical Technology S.A., 2557 Luxembourg (LU)
(72) Erfinder: Schröter, Christof, 76307 Karlsbad (DE); Schwaibold, Matthias, D-76228 Karlsruhe (DE)
(74) Vertreter: Marx, Thomas

(56) Entgegenhaltungen:
- US-A1- 2005 211 248
- US-A1- 2011 284 003
- US-B1- 6 439 229
- US-B2- 6 755 193
- US-B2- 8 528 556

## Beschreibung

Die Erfindung betrifft eine Vorrichtung zur Behandlung von Cheyne-Stokes-Atmung, periodischer Atmung und zentralen oder gemischten Apnoen.

Bei einigen Patienten ist der eigene Atemantrieb derart gestört, dass die Atmung periodisch an- und abschwillt. Gängige Verfahren zur Unterstützung erhöhen den Beatmungsdruck in Phasen der periodischen Atmung. US 2011/284003 A1 offenbart ein Beatmungsgerät, dass Schnarchen, Apnoen und Hypopnoen beispielsweise aus einer Analyse des Druck- oder Fluss-Signales detektiert. Die EP 1083953 beschreibt ein Verfahren zur Therapie von Cheyne-Stokes-Atmung. Dabei erfolgt eine fortlaufende Messung und Ermittlung des Volumens der Ventilation der letzten Minuten, wobei dieses gemittelt wird. Zudem wird ein Zielvolumen, typisch 90%, vorgegeben, welches sich aus der längerfristigen Ventilation der vorangegangenen Ventilation ergibt. Liegt eine Differenz zwischen dem Zielvolumen und dem aktuellen Volumen vor, so wird die Druckunterstützung durch das Beatmungsgerät angehoben. Im Ergebnis soll die aktuelle Atmung des Patienten derart durch einen Druckhub unterstützt werden, dass das Zielvolumen erreicht wird.

Nachteilig an diesem Verfahren ist, dass der Ansatz nicht berücksichtigt, dass sich die Atemlage verändern kann und die Atmung insgesamt eher chaotisch ist. Eine Veränderung der Atemlage bewirkt hier eine veränderte Zielventilation.

Wird beispielsweise das Zielvolumen über einen Zeitraum ermittelt, in dem der Patient intensiv geatmet hat - beispielsweise in einem Wachzustand - und wird dieses Zielvolumen mit der aktuellen gering ausgeprägten Atmung des Patienten im Schlaf verglichen, so würde der Algorithmus der EP 1083953 die Druckunterstützung über das Maß der natürlichen Atmung des Patienten im Schlaf anheben, weil ja das vorher ermittelte Zielvolumen auf der intensiven Atmung im Wachzustand beruht.

Ziel ist es daher, eine adaptive Druckunterstützung anzugeben, um Schwankungen beim Atemantrieb des Patienten auszugleichen, die jedoch die aktuelle natürliche Atmung des Patienten mit berücksichtigt.

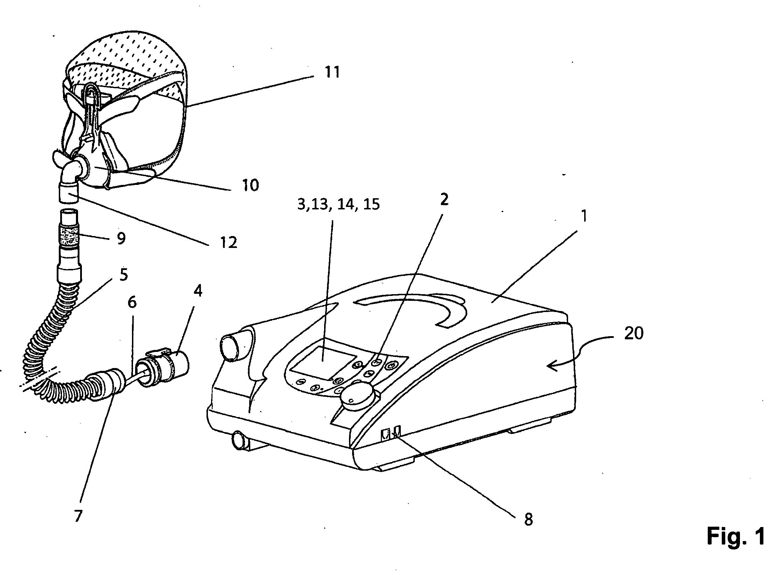

Beatmungsgerät mit einer steuerbaren Atemgasquelle zur Vorgabe von Beatmungsparametern bei der Atemgasversorgung eines Patienten mit periodischer Atmung
Mittel um ein aktuelles Maß eines Beatmungsparameters zu ermitteln
Speichermittel um ein Maß eines vorangegangenen Beatmungsparameters abrufbar zu bevorraten
Vergleichsmittel um ein Maß für die Änderung (Steigen / Fallen) des aktuellen Beatmungsparameters im Vergleich zum vorangegangenen Beatmungsparameter abzuleiten
Mittel um ein aktuelles Maß der Atmung zu ermitteln Speichermittel um ein Maß der vorangegangenen Atmung abrufbar zu bevorraten
Vergleichsmittel um ein Maß für die Änderung (Steigen / Fallen) der aktuellen Atmung im Vergleich zur vorangegangenen Atmung abzuleiten
Vergleichsmittel um ein Maß für die Änderung (Steigen / Fallen) der Atemanstrengung aus mindestens einem der Maße abzuleiten
einer Steuereinrichtung die zumindest einen Beatmungsparameter in Abhängigkeit von dem Maß steuert. Die erfindungsgemäße Aufgabe wird durch eine Vorrichtung mit den Merkmalen des Anspruchs 1 gelöst. Fig. 1 zeigt den grundsätzlichen Aufbau einer Vorrichtung zur Beatmung. Im Bereich eines Gerätegehäuses (1) des Beatmungsgerätes (20), mit einer Atemgasquelle im Geräteinnenraum, sind ein Bedienelement (2) und ein Bedien-und Informationssystem (3), bestehend aus einer Anzeige (13), einer berührungsempfindlichen Eingabeeinheit (15) mit zumindest einem Bedienfeld (14) angeordnet. Eine Steuereinheit (19) dient dazu, den Echtzeitbetrieb des Beatmungsgerätes zu steuern. Über eine Kopplung (4) wird ein Verbindungsschlauch (5) angeschlossen. Entlang des Verbindungsschlauches (5) kann ein zusätzlicher Druckmessschlauch (6) verlaufen, der über einen Druckeingangsstutzen (7) mit dem Gerätegehäuse (1) verbindbar ist. Zur Ermöglichung einer Datenübertragung weist das Gerätegehäuse (1) zumindest eine Schnittstelle (8) auf. Ein Anfeuchter (30) kann zudem adaptiert werden. Im Bereich einer dem Gerätegehäuse (1) abgewandten Ausdehnung des Verbindungsschlauches (5) ist ein Ausatmungselement (9) angeordnet. Ebenfalls kann ein Ausatemventil verwendet werden.

Fig. 1 zeigt darüber hinaus ein als Beatmungsmaske (10) ausgebildetes Patienten-Interface, das als Nasalmaske realisiert ist. Eine Fixierung im Bereich eines Kopfes eines Patienten kann über eine Kopfhaube (11) erfolgen. Im Bereich seiner dem Verbindungsschlauch (5) zugewandten Ausdehnung weist das Patienten-Interface (10) ein Kupplungselement (12) auf. Über die Schnittstelle (8) kann die Eingabe und / oder Ausgabe von Daten, wie beispielsweise Totraumvolumen, erfolgen. Die Schnittstellen können kabelgebunden, als Infrarot-Schnittstelle, als Bluetooth-Schnittstelle oder USB realisiert sein. Bevorzugt ist auch ein Kartenschacht vorgesehen. Die Schnittstelle (8) kann auch als LAN-Schnittstelle oder als sonstige Schnittstelle zur Anbindung an das Internet ausgeführt sein. Im Bereich eines Gerätegehäuses kann ein Sauerstoffzuschaltventil an die Vorrichtung zur Beatmung adaptiert werden. Es ist denkbar, das Atemgas zusätzlich mit Sauerstoff anzureichern, um die Patientenversorgung zu verbessern. Über die Schnittstelle (8) - beispielsweise als Kartenschacht oder USB ausgeführt - können auch therapiefremde Daten in das erfindungsgemäße Beatmungsgerät geladen werden beziehungsweise von diesem ausgeführt werden. So ist daran gedacht beispielsweise Fotos oder Videos mittels Speichermedien über die Schnittstelle (8) im Bereich der Anzeige darzustellen. Der Anwender muss - werden vom Gerät externe Speichermedien erkannt - eine Abfrage im Bedienfeld bestätigen, woraufhin die Daten wahlweise im Bereich des Beatmungsgerätes gespeichert oder ausgeführt werden. Das erfindungsgemäße Beatmungsgerät (20) ist so ausgelegt, dass es über einen Schlauch und ein Patienteninterface mit einem Patienten verbunden werden kann, um eine Beatmung bereitzustellen. Es umfasst eine Quelle für Atemgas, die beispielsweise als ein Elektromotor mit Gebläserad ausgebildet ist, und zumindest eine SensorEinrichtung (21) zur Ermittlung von Druck und/oder Fluss und/oder Volumen des Atemgases, sowie eine Steuereinheit (19), die so ausgelegt ist, dass sie für jeden Atemzyklus auf der Basis eines vorbestimmten Wertes für den Patienten und/oder auf Basis von Messsignalen für die Parameter Druck und/oder Flow und/oder Volumen einen Atemgasdruck bestimmt und die Quelle für Atemgas derart reguliert, dass der Atemgasdruck erzeugt wird.

Die Steuereinheit (19) ist ferner so ausgelegt, dass sie den aktuellen Druck und/oder Flow und/oder das Volumen von Atemgas bestimmt und den aktuellen Wert über das mit der Steuereinheit verbundene Bedien- und Informationssystem (3) darstellt. Die Steuereinheit (19) ist außerdem so ausgelegt, dass sie bezogen auf einen oder mehrere Parameter mittels eines Statistikmoduls (23) Trendveränderungen über eine Zeitdauer bestimmt, wobei die Trendveränderungen im Speicher (22) abgelegt werden oder auf der Anzeige angezeigt werden können.

Weiterhin vergleicht die Steuereinheit (19) solche ParameterWerte, die von einem Benutzer vorgegeben wurden, beispielsweise obere und untere Druckgrenzen oder eine maximal tolerierbare Anzahl von Apnoen pro Zeiteinheit, oder eine maximal tolerierbare Leckage, mit den aktuellen Werten und generiert eine Benutzer-Information zu Abweichungen von der Vorgabe. Die Benutzerinformation wird bevorzugt über das Bedien- und Informationssystem (3) im Bereich der Anzeige (14) graphisch visualisiert.

So werden beispielsweise aus dem gemessenen Atemfluss über eine Abnahme des Atem(zeit)volumens für eine Zeitdauer von mindestens 3 Sekunden Apnoen und Hypopnoen erkannt. Zusätzlich wird über Druck- und Flowschwankungen Schnarchen erkannt, sowie über die inspiratorische Flowkontur Flattening erkannt. Daraus werden durch das Statistikmodul (23) für jede ausreichend lange nächtliche Therapie Indizes berechnet, nämlich: AHI (= Anzahl Apnoen + Hypopnoen pro artefaktfreier Therapiedauer), RDI (= Anzahl aller respiratorischen Ereignisse pro artefaktfreier Therapiedauer), Anteil Atemzüge mit Flattening, Anteil Atemzüge mit Schnarchen. Bevorzugt werden auch Daten ermittelt, die auf das Nutzungsverhalten oder auf die Nutzungsdauer des Gerätes durch den Patienten schließen lassen. Diese Daten werden täglich, wöchentlich oder monatlich durch das Statistikmodul ermittelt und gespeichert. Bedarfsweise werden diese Nutzungsdaten, gegebenenfalls zusammen mit einer Gerätekennung, gerätebezogenen Daten und/oder Patientendaten bzw.-kennung gespeichert.

Gerätebezogene Daten können beispielsweise Gerätetyp, Seriennummer, Firmware-Version, Elektronik-Version, Betriebsstunden von einer oder mehreren Komponenten sowie Fehlerspeicher sein. Patientenbezogene Daten können beispielsweise Name, Ident-Nr., Geburtsdatum, Versicherungsnummer, Verordnungsnummer oder Aufenthaltsort beinhalten.

Patienteneingaben wie beispielsweise subjektives Empfinden, Therapienebenwirkungen, Schweregrad der Erkrankung oder Medikation können ebenfalls gespeichert und dargestellt werden.

Des Weiteren können auch Geräteeinstellungen wie Druck(grenzen), Frequenz, Ti/T, Targetvolumen, Triggerstufe, Comfort-Settings, Werte zu Luft-Temperatur und -Feuchte sowie Befeuchter-Stufe ebenso wie Nutzungsdaten wie beispielsweise Anzahl Stunden an einzelnen Tagen oder innerhalb von bestimmten Zeiträumen, mittlere Stunden, minimale / maximale Stunden, Anteil Tage mit mehr Nutzung als einem Schwellwert, Nutzungsinformation zum Befeuchter, etc. gespeichert und dargestellt werden.

Neben den bereits beschriebenen Indizes wie AHI und RHI können weitere Therapiequalitäts-Indizes wie Schnarch-Index, RERA-Index, oAHI, zAHI, Index für periodische Atmung / CS-Atmung, Volumen-Index, Schlafqualitäts-Index, Sauerstoff-Index, CO2-Index, Atemarbeits-Index, Trigger-Qualitäts-Index, Anteil Atemzüge mit/ohne Spontanatmung bzw. an bestimmten Druckgrenzen, Perzentile von Druck, Frequenz, Volumen, Obstruktionsgrad, Ti/T oder ähnlichen Parametern, Blutdruck-Index, Blutzucker-Index, Herzfrequenz-Index mittels Statistikmodul ermittelt, gespeichert und dargestellt werden.

Das Bedien- und Informationssystem (3) für ein Beatmungsgerät (20) mit einer beleuchteten oder hinterleuchteten Anzeige (13) zur Darstellung von Bedienfeldern (14) oder Informationen für den Anwender und einer beispielsweise berührungsempfindlichen Eingabeeinheit (15), die auch als mechanische Eingabeeinheit ausgebildet sein kann, in räumlicher Nähe zum dargestellten Bedienfeld (14). In einer konkreten Ausführungsform handelt es sich beispielsweise um eine Mensch-Maschine-Schnittstelle in Form eines sogenannten Touchscreens, wobei der Fachmann unterschiedliche Typen kennt, die alle als Bestandteil des erfindungsgemäßen Bedien- und Informationssystems (3) in Frage kommen. Im Bereich der Anzeige werden zumindest ein erstes Bedienfeld und ein zweites Bedienfeld dargestellt. Die Steuereinheit (19) ist ausgestaltet, das Menü auf der Anzeige (13) darzustellen. Eine Verarbeitungseinheit (18) welche mit der Anzeige (13) und der berührungsempfindlichen Eingabeeinheit (15) gekoppelt ist, ist ausgestaltet eine Bedienung des Bedienfeldes (14) über die Eingabeeinheit (15) zu erfassen und in Abhängigkeit davon eine Funktion des Menüs über die Steuereinheit (19) anzusteuern.

Der Speicher (22) kann verwendet werden, um aktuelle Einstellungen, den Systemstatus, Patientendaten und Beatmungssteuerungs-software zu speichern, die durch die Steuereinheit ausgeführt wird. Die Steuereinheit kann außerdem mit einer Speichereinrichtung verbunden sein, wie zum Beispiel einem mittels einer Batterie gepufferten Speicher, einer Speicherkarte, einer Festplatte, einem Diskettenlaufwerk, einem Magnetbandlaufwerk oder irgendeinem anderen Speichermedium zum Speichern von Patientendaten und zugehörigen Beatmungsgerät-Betriebsparametern. Die Steuereinheit akzeptiert Eingaben, die vom Bediensystem empfangen werden, um das Beatmungsgerät (20) zu steuern. Das Beatmungsgerät kann außerdem Status-Indikatoren, eine Anzeige, um Patientendaten und Beatmungsgerät-Einstellungen zu speichern, sowie einen Audio-Generator aufweisen, um hörbare Angaben bezüglich des Status des Beatmungsgeräts zur Verfügung zu stellen.

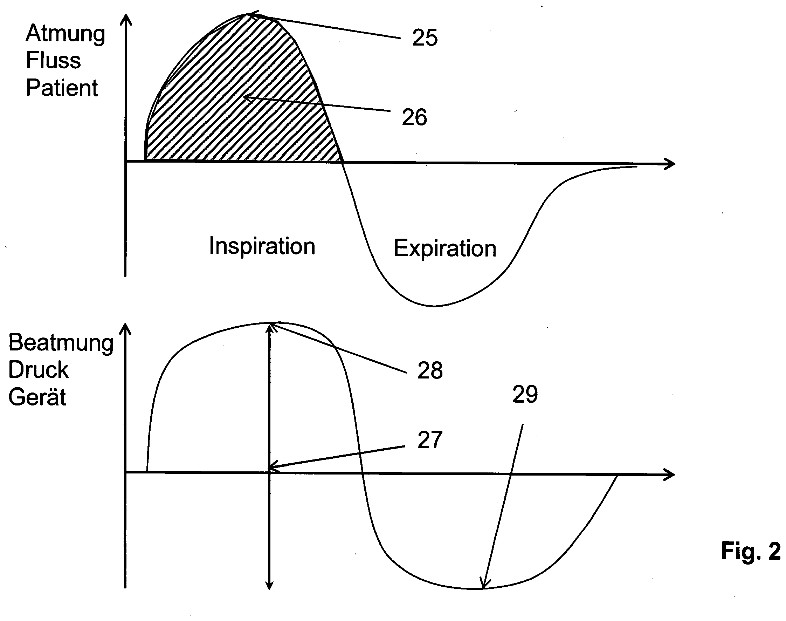

Figur 2 zeigt oben den Verlauf der Atmung des Patienten. Hier wurde mit Hilfe eines Fluss-Sensors der Fluss ermittelt und aufgezeichnet. Eingezeichnet sind der Spitzenfluss (25), etwa zur Mitte der Inspiration, und das Tidalvolumen (26), welches der Patient in der Inspiration einatmet. Das Minutenvolumen ist das inspiratiorische Volumen pro Minute und kann gemäß Figur 2 zu jedem Zeitpunkt berechnet werden. Figur 2 zeigt unten den Verlauf der Druckunterstützung (27) durch das Beatmungsgerät. Hier wurde mit Hilfe eines Druck-Sensors der Druck ermittelt und aufgezeichnet. Eingezeichnet sind der IPAP (28), welcher etwa zur Mitte der Inspiration erreicht wird. Außerdem ist der expiratorische Druck EPAP (29) eingezeichnet. Die Differenz aus IPAP und EPAP ist die Druckunterstützung (27). Die Beatmung mit Druckunterstützung (PDIFF) erhöht, durch atemphasensynchrone Applikation der Drücke, das Tidalvoumen und/oder Minutenvolumen der Patientenatmung und verbessert somit die Ventilation der Lunge.

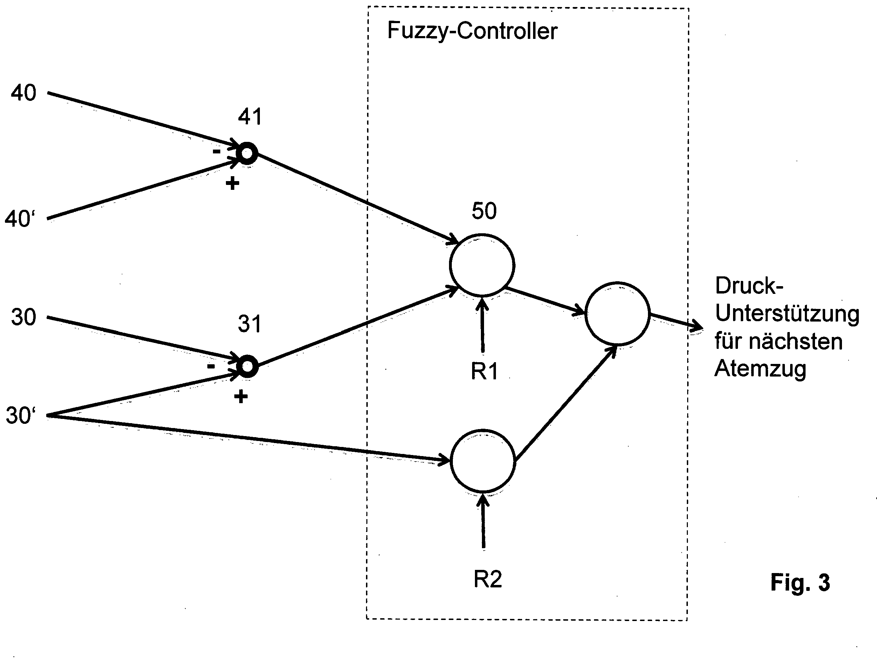

Figur 3 zeigt: Ein Beatmungsgerät mit einer steuerbaren Atemgasquelle zur Vorgabe von Beatmungsparametern bei der Atemgasversorgung eines Patienten mit periodischer Atmung, Mittel (21, 22), um ein aktuelles Maß eines Beatmungsparameters (30') zu ermitteln Speichermittel (22), um ein Maß eines vorangegangenen Beatmungsparameters (30) abrufbar zu bevorraten, Vergleichsmittel um ein Maß für die Änderung (31) (Steigen / Fallen) des aktuellen Beatmungsparameters im Vergleich zum vorangegangenen Beatmungsparameter abzuleiten, Mittel (21) um ein aktuelles Maß der Atmung (40') zu ermitteln, Speichermittel (22) um ein Maß der vorangegangenen Atmung (40) abrufbar zu bevorraten, Vergleichsmittel um ein Maß (41) für die Änderung (Steigen / Fallen) der aktuellen Atmung im Vergleich zur vorangegangenen Atmung abzuleiten, Vergleichsmittel um ein Maß für die Änderung (Steigen / Fallen) der Atemanstrengung (50) aus mindestens einem der Maße (31, 41) abzuleiten, einer Steuereinrichtung die zumindest einen Beatmungsparameter in Abhängigkeit von dem Maß (50) steuert.
Das Beatmungsgerät ermittelt ein Maß für die aktuelle Atmung, indem mittels eines Fluss- und/oder Druck-Sensors (21) die Atmung des Patienten aufgezeichnet wird. Das Maß für die aktuelle Atmung (40') wird aus dem Druck und/oder Flussverlauf ermittelt beispielsweise als Spitzenfluss (25) und/oder Volumen und/oder Zeitvolumen und/oder aktueller Fluss und/oder Tidalvolumen (26) und/oder relatives Volumen und/oder Minutenvolumen. Das Maß für die aktuelle Atmung (40') bezieht sich dabei bevorzugt auf nur einen Atemzug, nämlich den letzten. Das Beatmungsgerät speichert dieses Maß der aktuellen Atmung in einem Speicher (22). Da das Beatmungsgerät im Betrieb somit fortlaufend, nämlich mit jedem Atemzug, ein Maß der aktuellen Atmung (40') aufzeichnet, befinden sich im Speicher chronologisch die Werte der letzten und vorletzten Atemzüge. Aus den Werten der Maße der Atmung der letzten (40") und vorletzten Atemzüge (40) ermittelt das Beatmungsgerät, durch Verrechnung, ein Maß für die Änderung (41) (Steigen / Fallen) der Atmung.

Das Beatmungsgerät ermittelt ein Maß für die aktuelle Beatmung (beispielsweise Druckunterstützung, Druckhub oder Frequenz), indem mittels eines Fluss- und/oder Druck-Sensors die Beatmung durch das Gerät aufgezeichnet wird oder die angewendeten Werte verwendet werden. Das Maß für die aktuelle Beatmung (30') wird beispielsweise aus dem Druck und/oder Flussverlauf ermittelt als Spitzenfluss und/oder Volumen und/oder Zeitvolumen und/oder aktueller Fluss und/oder Tidalvolumen und/oder relatives Volumen und/oder Minutenvolumen. Das Maß für die aktuelle Beatmung (30') bezieht sich dabei bevorzugt auf nur einen Atemzug, nämlich den letzten.

Das Beatmungsgerät speichert dieses Maß der aktuellen Beatmung in einem Speicher (22). Da das Beatmungsgerät im Betrieb somit fortlaufend, nämlich mit jedem Atemzug, ein Maß der aktuellen Beatmung (30') aufzeichnet, befinden sich im Speicher chronologisch die Werte der letzten und vorletzten Atemzüge. Aus den Werten der Maße der Beatmung der letzten (30') und vorletzten (30) Atemzüge ermittelt das Beatmungsgerät, durch Verrechnung, ein Maß für die Änderung (31) (Steigen / Fallen) der Beatmung (Druckunterstützung).

Aus den Werten der Maße Änderung (Steigen / Fallen) der Beatmung (31) und Änderung (Steigen / Fallen) der Atmung (41) ermittelt das Beatmungsgerät, durch Verrechnung, ein Maß für die Änderung (Steigen / Fallen) der Atemanstrengung (50) des Patienten. Beispielsweise ermittelt das Beatmungsgerät so die Atemanstrengung (50) für den vorletzten Atemzug und die für den letzten Atemzug und vergleicht diese. Die Atemanstrengung (50) des Patienten dient als erster Referenzwert (R1). Beispielsweise eine sich nicht ändernde Atemanstrengung (50) des Patienten dient als erster Referenzwert (R1).

Die Beatmung (31, beispielsweise als gleichbleibende Druckunterstützung) des Patienten dient als zweiter Referenzwert (R2). Beispielsweise eine sich nicht ändernde Beatmung (31) des Patienten dient als zweiter Referenzwert (R2). Ändert sich die Atemanstrengung nicht und ändert sich die Beatmung nicht, so bleibt die Druckunterstützung für den nächsten Atemzug unverändert. Das Beatmungsgerät kann beide Referenzwerte (R1 und R2) für die Regelung der Druckunterstützung für den nächsten Atemzug berücksichtigen. Alternativ kann das Beatmungsgerät auch nur R1 berücksichtigen.

Auf eine Änderung des Wertes der Atemanstrengung (50) des Patienten reagiert das Beatmungsgerät mit einer Änderung der Beatmung, also beispielsweise der Druckunterstützung. Typischerweise wird die Druckunterstützung, bevorzugt proportional zur Änderung der Atemanstrengung, angepasst. Sinkt die Atemanstrengung (50) des Patienten reagiert das Beatmungsgerät mit einer Anhebung der Druckunterstützung. beispielsweise werden IPAP und EPAP verändert, sodass der Druckhub größer wird. Alternativ kann auch nur der IPAP angehoben werden. Ebenfalls kann auch nur der EPAP abgesenkt werden. Typischerweise wird die Druckunterstützung, bevorzugt proportional zur Änderung der Atemanstrengung, angepasst.

Die Beatmung (z. B. Druckhub oder Frequenz) wird beispielsweise antizyklisch zum Wert der Atemanstrengung des Patienten gesteuert. Beispielsweise im Bereich 0,5 - 4 hPa mehr Beatmungs-Druck bei einem Abfall der Atmung um 20 %.

Die antizyklische Beatmung wird verstärkt oder abgeschwächt je nach vorangegangener Hyperventilation. Je größer die Hyperventilation, desto stärker die Verstärkung.

Als Maß für die Hyperventilation gelten aufeinanderfolgende Atemzüge mit erhöhtem Volumen. Suchfenster ist etwa die Dauer eines CS-Atemzyklus (also die zurückliegenden ca. 90s), danach ist der Pegel der Verstärkung weitgehend abgeklungen.

Die resultierende Änderung der Beatmung wird vor Anwendung am Patienten noch begrenzt, beispielsweise auf maximal 4 hPa Unterschied des Druckhubes zwischen 2 Atemzügen.

Die Beatmung kann zwischen 2 Atemzügen anhand der Auswertung des letzten Atemzuges erfolgen, oder direkt innerhalb des aktuellen Atemzuges unter Auswertung des augenblicklichen Atmungsverlaufes.
Nimmt der Quotient aus aktuellem Volumen und Normvolumen (rAMV) (wird alle 2 Minuten aktualisiert)) ab, wird PDIFF erhöht und umgekehrt. Die Stärke der PDIFF-Änderung hängt dabei nicht nur von der Stärke der rAMV-Änderung ab, sondern zusätzlich vom vorausgehenden Hyperventilationspegel: Je höher dieser ist, desto dynamischer die Reaktion.

Die Hyperventilation kann bei CS-Patienten als der Auslöser der Atem-Instabilität gesehen werden. Bei einer Hyperventilation wird daher nicht gewartet, bis das rAMV des Patienten unter sein "Targetvolumen" (das wären ca. 100% rAMV) fällt, sondern wird durch steigende PDIFF die Beginnende Hyperventilation aufgefangen, wenn das rAMV zu fallen beginnt. Die Stärke des Hyperventilations-Peaks kann als Maß für die Ausprägung der CS-Atmung betrachtet werden. Deshalb wird dieser Wert als Verstärkungsfaktor genutzt. Somit wird eine Reaktion auf die Periodik mit adaptiver PDIFF-Dynamik erzielt. Das führt z.B. auch dazu, dass in der Einschlafphase, wenn das Volumen ganz physiologisch kontinuierlich etwas absinkt nicht mit PDIFF-Anhebung der Patienten wieder geweckt wird, weil der Volumenreduktion keine Hyperventilation vorangeht.

Für (E)EPAP, Druckhub und Frequenz sind Regelgrenzen einstellbar. Für den Druckhub ist vorzugsweise zusätzlich ein Wert für die basale Unterstützung der Atemmuskulatur auswählbar (Druckhub bei Normalatmung).
In Phasen mit weitgehend stabiler Atmung (Änderung gering) ändert der Regler den Druckhub schrittweise hin zu der vorgewählten basalen Unterstützung. Phasen mit weitgehend stabiler Atmung (Änderung gering) zeichnen sich dadurch aus, dass die Atemanstrengung von Atemzug zu Atemzug um weniger als 20 %, bevorzugt um weniger als 10 %, besonders bevorzugt um weniger als 5 % abweicht. Phasen mit weitgehend stabiler Atmung (Änderung gering) zeichnen sich alternativ auch dadurch aus, dass die Druckunterstützung von Atemzug zu Atemzug um weniger als 20 %, bevorzugt um weniger als 10 %, besonders bevorzugt um weniger als 5 % abweicht.

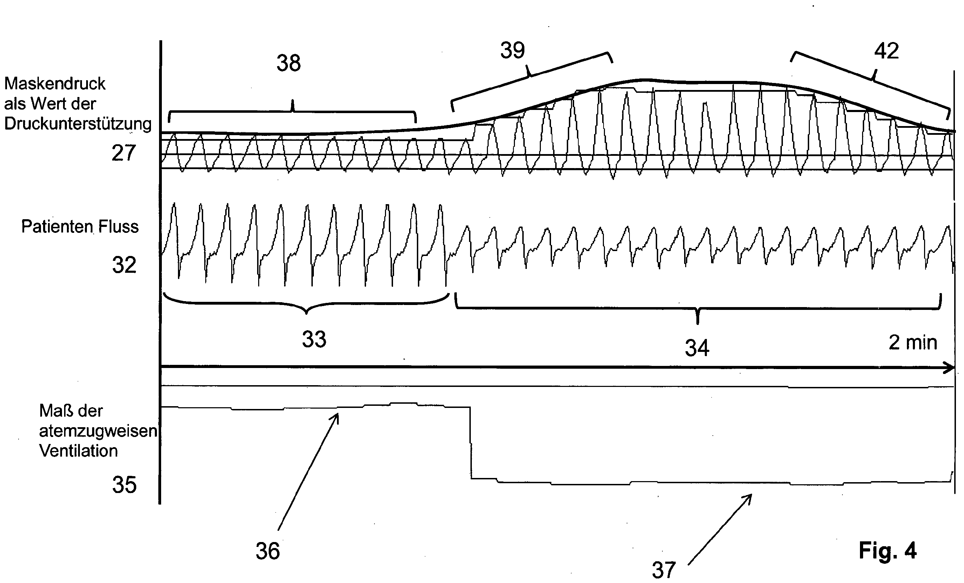

Figur 4 zeigt: Das Beatmungsgerät ermittelt ein Maß für die aktuelle Atmung, indem mittels eines Fluss-Sensors die Atmung des Patienten aufgezeichnet wird ("Patienten Fluss", 32).
Das Beatmungsgerät ermittelt zudem ein Maß der "atemzugweisen Ventilation" (35) für die aktuelle Atmung aus dem Druck und/oder Flussverlauf und/oder Volumen und/oder Zeitvolumen und/oder Tidalvolumen und/oder Minutenvolumen.

Das Beatmungsgerät speichert dieses Maß der "atemzugweisen Ventilation" (35) in einem Speicher (22). Beispielsweise wird die "atemzugweisen Ventilation" (35) als Indexwert zu 100% betrachtet und gespeichert, wobei 100% (36) bedeutet, dass der Wert langfristig stabil war.

Man erkennt aus dem Vergleich der mittleren Kurve "Patienten Fluss" (32) mit der unteren Kurve "atemzugweise Ventilation" (35), dass bei abnehmendem Patientenfluss (34) auch die atemzugweise Ventilation (37) abnimmt. In diesem Beispiel von 100 auf 50%. Gemäß dem in der Beschreiung zu Fig. 3 erläuterten Verfahren, reagiert das Beatmungsgerät bei gleichbleibender Ventilation (36) mit gleichbleibender Druckunterstützung (38). Nimmt die Ventilation ab (37), weil beispielsweise der Patientenfluss abnimmt, so erhöht das Beatmungsgerät die Druckunterstützung (39), indem der IPAP und/oder der EPAP angepasst werden, so lange, bis eine langfristig (über zumindest 2 Atemzüge) stabile Ventilation ermittelt wird. Ist die Ventilation wieder stabil, so nimmt das Beatmungsgerät die Druckunterstützung (42) zurück, indem der IPAP und/oder der EPAP angepasst werden.

## Patentansprüche

1. Beatmungsgerät mit einer steuerbaren Atemgasquelle zur Vorgabe von Beatmungsparametern bei der Atemgasversorgung eines Patienten mit periodischer Atmung umfassend:
einen Verbindungsschlauch zu einem Patienteninterface ein Statistikmodul (23)
Mittel (21, 22) um ein aktuelles Maß eines Beatmungsparameters (30) zu ermitteln
Speichermittel (22) um ein Maß eines vorangegangenen Beatmungsparameters (30) abrufbar zu bevorraten
Vergleichsmittel um ein Maß (31) für die Änderung des aktuellen Beatmungsparameters (30') im Vergleich zum vorangegangenen Beatmungsparameter (30) abzuleiten
Mittel (21) um ein aktuelles Maß der Atmung (40") zu ermitteln Speichermittel (22) um ein Maß der vorangegangenen Atmung (40) abrufbar zu bevorraten
Vergleichsmittel um ein Maß (41) für die Änderung der aktuellen Atmung im Vergleich zur vorangegangenen Atmung abzuleiten Vergleichsmittel um ein Maß (50) für die Änderung der Atemanstrengung aus mindestens einem der Maße (31, 41) abzuleiten eine Steuereinrichtung (19) die zumindest einen Beatmungsparameter in Abhängigkeit von dem Maß (50) steuert **dadurch gekennzeichnet, dass**
die Steuereinheit (19) bezogen auf einen oder mehrere Beatmungsparameter (30, 30') mittels des Statistikmoduls (23)
Trendveränderungen über eine Zeitdauer bestimmt, wobei die Trendveränderungen im Speicher (22) abgelegt werden , wobei das Beatmungsgerät ein Maß für die aktuelle Atmung (40") ermittelt,
indem mittels eines Fluss- und/oder Druck-Sensors (21) die Atmung des Patienten aufgezeichnet wird, wobei das Maß für die aktuelle Atmung (40") aus dem Druck und/oder Flussverlauf ermittelt wird und dieses Maß der aktuellen Atmung (40") in dem Speicher (22) gespeichert wird, wobei aus den Werten der Maße der Atmung der letzten (40") und vorletzten Atemzüge (40), durch Verrechnung, ein Maß für die Änderung (41) der Atmung ermittelt wird, wobei
ein Maß für die aktuelle Beatmung ermittelt wird, indem mittels des Fluss- und/oder Druck-Sensors (21) die Beatmung durch das Gerät aufgezeichnet wird und
dieses Maß der aktuellen Beatmung in dem Speicher (22) gespeichert wird und aus den Werten der Maße der Beatmung der letzten (30') und vorletzten (30) Atemzüge durch Verrechnung, ein Maß für die Änderung (31) der Beatmung ermittelt, wobei aus den Werten der Maße Änderung der Beatmung (31) und Änderung der Atmung (41), durch Verrechnung, ein Maß für die Änderung der Atemanstrengung (50) des Patienten ermittelt wird, wobei die Steuereinrichtung (19), als Beatmungsparameter, zumindest den IPAP-Druck und/oder den EPAP-Druck für den folgenden Atemzug in Abhängigkeit von der Änderung der Atemanstrengung (50) der vorangegangenen Atemzüge steuert.

2. Beatmungsgerät nach Anspruch 1, **dadurch gekennzeichnet, dass** die Steuereinrichtung zumindest einen Beatmungsparameter in Abhängigkeit von dem Maß (50) steuert, mit dem Ziel eine Steigung von 0 für das Maß (50) zu erreichen.

3. Beatmungsgerät nach Anspruch 1 oder 2, **dadurch gekennzeichnet, dass** die Steuereinrichtung die zumindest einen Beatmungsparameter in Abhängigkeit von dem Maß (50) steuert, mit dem Ziel eine Steigung von 0 für das Maß (30) zu erreichen.

4. Beatmungsgerät nach zumindest einem der vorhergehenden Ansprüche, **dadurch gekennzeichnet, dass** die Steuereinrichtung in Phasen mit weitgehend stabiler Atmung, bei denen die Atemanstrengung von Atemzug zu Atemzug um weniger als 20 % abweicht, den Druckhub, als Differenz zwischen IPAP- und EPAP-Druck, schrittweise hin zu einer vorgewählten basalen Unterstützung führt.

5. Beatmungsgerät nach zumindest einem der vorhergehenden Ansprüche, **dadurch gekennzeichnet, dass** bei Veränderungen der Ventilation im Verlaufe der Nacht die Druckunterstützung auf Atemzug-zu-Atemzug Basis angepasst wird.

6. Beatmungsgerät nach zumindest einem der vorhergehenden Ansprüche, **dadurch gekennzeichnet, dass** Aufzeichnungen der Druckkurve und/oder Flusskurve und/oder Atemfrequenz der natürlichen Atmung des Patienten zumindest zeitweise zur Modulation der Druckunterstützung verwendet werden.

7. Beatmungsgerät nach zumindest einem der vorhergehenden Ansprüche, **dadurch gekennzeichnet, dass** die Aufzeichnung der Druckkurve und/oder Flusskurve und/oder Atemfrequenz der natürlichen Atmung des Patienten zumindest zeitweise zur Berechnung einer Sicherheitsfrequenz für die Beatmung genutzt wird.

8. Beatmungsgerät nach zumindest einem der vorhergehenden Ansprüche, **dadurch gekennzeichnet, dass** der endexspiratorische Druck (EPAP) von 2 bis auf 15 cm H2O eingestellt werden kann.

9. Beatmungsgerät nach zumindest einem der vorhergehenden Ansprüche, **dadurch gekennzeichnet, dass** verschiedene Druckprofile für Einatmung und Ausatmung anwendbar sind.

10. Beatmungsgerät nach zumindest einem der vorhergehenden Ansprüche, **dadurch gekennzeichnet, dass** Obstruktionen durch FOT und/oder Flattening und/oder Schnarchen und/oder Apnoe und/oder eine Flowantwort auf mandatorische Beatmung erkannt werden.

11. Beatmungsgerät nach zumindest einem der vorhergehenden Ansprüche, **dadurch gekennzeichnet, dass** der Cardiac Output oder Blutdruck oder Herzfrequenz oder SpO2 oder CO2 über ein adaptierbares Modul ermittelt werden und die Meßgrößen von Cardiac Output oder Blutdruck oder Herzfrequenz oder SpO2 oder CO2 für die Berechnung der Druckunterstützung verwendet werden.

12. Beatmungsgerät nach zumindest einem der vorhergehenden Ansprüche, **dadurch gekennzeichnet, dass** die Regelung der Leistung eines mit dem Beatmungsgerät verbundenen Anfeuchters in Abhängigkeit von dem Maß der Druckunterstützung erfolgt.

13. Beatmungsgerät nach zumindest einem der vorhergehenden Ansprüche, **dadurch gekennzeichnet, dass** in einer initialen Gewöhnungsphase eine geringere als die als optimal berechnete Druckunterstützung, appliziert wird.

## Claims

1. Ventilator comprising a controllable respiratory gas source for predetermining ventilation parameters in the respiratory gas supply of a patient with periodic respiration, comprising:
a connecting tube to a patient interface,
a statistics module (23),
means (21, 22) for establishing a current measure of a ventilation parameter (30),
storage means (22) for storing a measure of a preceding ventilation parameter (30) in a recallable manner,
comparison means for deriving a measure (31) for the change in the current ventilation parameter (30') compared to the preceding ventilation parameter (30),
means (21) for establishing a current measure of the respiration (40'),
storage means (22) for storing a measure of the preceding respiration (40) in a recallable manner,
comparison means for deriving a measure (41) for the change in the current respiration compared to the preceding respiration,
comparison means for deriving a measure (50) for the change in the respiratory exertion from at least one of the measures (31, 41),
a control apparatus (19) which controls at least one ventilation parameter dependent on the measure (50),
**characterized in that** the control unit (19), by way of the statistics module (23), determines changes in the trend in relation to one or more ventilation parameters (30, 30') over a period of time, wherein the changes in the trend are stored in the memory (22), wherein the ventilator establishes a measure for the current respiration (40') by virtue of the respiration of the patient being recorded by means of a flow and/or pressure sensor (21), wherein the measure for the current respiration (40') is established from the pressure and/or flow profile, and this measure of the current respiration (40') is stored in the memory (22), wherein, by calculation, a measure for the change (41) in the respiration is established from the values of the measures of the respiration of the last (40') and second-to-last breaths (40), wherein a measure is established for the current ventilation by virtue of the ventilation being recorded by the instrument by means of the flow and/or pressure sensor (21) and
this measure of the current ventilation is stored in the memory (22) and, by calculation, a measure is established for the change (31) in the ventilation from the values of the measures of the respiration of the last (30') and second-to-last (30) breaths, wherein,
by calculation, a measure is established for the change in the respiratory exertion (50) of the patient from the values of the measures of the change in the ventilation (31) and change in the respiration (41), wherein the control apparatus (19), as ventilation parameter, controls at least the IPAP pressure and/or the EPAP pressure for the subsequent breath dependent on the change in the respiratory exertion (50) in the preceding breaths.

2. Ventilator as claimed in Claim 1, **characterized in that** the control apparatus controls at least one ventilation parameter dependent on the measure (50) with the object of achieving a gradient of 0 for the measure (50).

3. Ventilator as claimed in Claim 1 or 2, **characterized in that** the control apparatus controls the at least one ventilation parameter dependent on the measure (50) with the object of achieving a gradient of 0 for the measure (30).

4. Ventilator as claimed in at least one of the preceding claims, **characterized in that** the control apparatus leads the pressure lift, i.e. the difference between the IPAP pressure and EPAP pressure, to a preselected basal support in a step-by-step manner in phases with largely stable respiration, in which the respiratory exertion deviates by less than 20% from breath to breath.

5. Ventilator as claimed in at least one of the preceding claims, **characterized in that** the pressure support is adapted on a breath-by-breath basis in the case of changes in the ventilation during the night.

6. Ventilator as claimed in at least one of the preceding claims, **characterized in that** recordings of the pressure curve and/or flow curve and/or respiratory frequency of the natural respiration of the patient are used, at least from time to time, for modulating the pressure support.

7. Ventilator as claimed in at least one of the preceding claims, **characterized in that** the recording of the pressure curve and/or flow curve and/or respiratory frequency of the natural respiration of the patient is used, at least from time to time, for calculating a safety frequency for the ventilation.

8. Ventilator as claimed in at least one of the preceding claims, **characterized in that** the expiratory positive airway pressure (EPAP) can be set from 2 to 15 cm H2O.

9. Ventilator as claimed in at least one of the preceding claims, **characterized in that** different pressure profiles are appliable for inspiration and expiration.

10. Ventilator as claimed in at least one of the preceding claims, **characterized in that** obstructions are identified by FOT and/or flattening and/or snoring and/or apnea and/or a flow response to mandatory ventilation.

11. Ventilator as claimed in at least one of the preceding claims, **characterized in that** the cardiac output or blood pressure or cardiac frequency or SpO2 or CO2 are established by means of an adaptable module and the measured variables of cardiac output or blood pressure or cardiac frequency or SpO2 or CO2 are used for calculating the pressure support.

12. Ventilator as claimed in at least one of the preceding claims, **characterized in that** the closed-loop control of the power of a humidifier connected to the ventilator is carried out dependent on the measure of the pressure support.

13. Ventilator as claimed in at least one of the preceding claims, **characterized in that** a pressure support that is lower than the ideal calculated one is applied during an initial accustoming phase.

## Revendications

1. Appareil de ventilation comprenant une source de gaz respiratoire commandable, destiné à prédéfinir des paramètres de ventilation lors de l'alimentation en gaz respiratoire d'un patient dont la respiration est périodique, comprenant :
un tuyau de liaison vers une interface de patient d'un module statistique (23),
des moyens (21, 22) pour déterminer une mesure actuelle d'un paramètre de ventilation (30),
des moyens de mémoire (22) pour mettre en réserve de manière consultable une mesure d'un paramètre de ventilation précédent (30),
des moyens de comparaison pour dériver une mesure (31) pour la modification du paramètre de ventilation actuel (30') en comparaison du paramètre de ventilation précédent (30),
des moyens (21) pour déterminer une mesure actuelle de la respiration (40'),
des moyens de mémoire (22) pour mettre en réserve de manière consultable une mesure de la respiration précédente (40),
des moyens de comparaison pour dériver une mesure (41) pour la modification de la respiration actuelle en comparaison de la respiration précédente,
des moyens de comparaison pour dériver une mesure (50) pour la modification de l'effort respiratoire à partir d'au moins l'une des mesures (31, 41),
un dispositif de commande (19) qui commande au moins un paramètre de ventilation en fonction de la mesure (50),
**caractérisé en ce que**
l'unité de commande (19) détermine des variations de tendance sur une période donnée en référence à un ou plusieurs paramètres de ventilation (30, 30') au moyen du module statistique (23),
les variations de tendance étant stockées dans la mémoire (22), l'appareil de ventilation déterminant une mesure pour la respiration actuelle (40') **en ce que** la respiration du patient est enregistrée au moyen d'un capteur de débit et/ou de pression (21), la mesure pour la respiration actuelle (40') étant déterminée à partir de la courbe de pression et/ou de débit et cette mesure de la respiration actuelle (40') étant mise en mémoire dans la mémoire (22), une mesure pour la modification (41) de la respiration étant déterminée par calcul à partir des valeurs des mesures de la respiration du dernier (40') et de l'avant-dernier (40) cycle respiratoire,
une mesure pour la ventilation actuelle étant déterminée en enregistrant la ventilation à travers l'appareil au moyen d'un capteur de débit et/ou de pression (21) et
cette mesure de la ventilation actuelle étant mise en mémoire dans la mémoire (22) et une mesure pour la modification (31) de la ventilation étant déterminée à partir des valeurs des mesures de la ventilation du dernier (30') et de l'avant-dernier (30) cycle respiratoire,
une mesure pour la modification de l'effort respiratoire (50) du patient étant déterminée par calcul à partir des valeurs des mesures de la modification de la ventilation (31) et de la modification de la respiration (41),
le dispositif de commande (19) commandant comme paramètre de ventilation au moins la pression PIP et/ou la pression PEP pour le cycle respiratoire suivant en fonction de la modification de l'effort respiratoire (50) des cycles respiratoires précédents.

2. Appareil de ventilation selon la revendication 1, **caractérisé en ce que** le dispositif de commande commande au moins un paramètre de ventilation en fonction de la mesure (50) avec pour objectif d'atteindre une pente de 0 pour la mesure (50).

3. Appareil de ventilation selon la revendication 1 ou 2, **caractérisé en ce que** le dispositif de commande commande l'au moins un paramètre de ventilation en fonction de la mesure (50) avec pour objectif d'atteindre une pente de 0 pour la mesure (30) .

4. Appareil de ventilation selon au moins l'une des revendications précédentes, **caractérisé en ce que** le dispositif de commande, dans les phases où la respiration est en grande partie stable, lors desquelles l'effort respiratoire d'un cycle respiratoire à l'autre varie de moins de 20 %, amène pas à pas l'excursion de pression, en tant que différence entre la pression PIP et PEP, jusqu'à une assistance basale présélectionnée.

5. Appareil de ventilation selon au moins l'une des revendications précédentes, **caractérisé en ce qu'**en présence de modifications de la ventilation au cours de la nuit, l'assistance en pression est adaptée sur la base d'un cycle respiratoire à l'autre.

6. Appareil de ventilation selon au moins l'une des revendications précédentes, **caractérisé en ce que** les enregistrements de la courbe de pression et/ou de la courbe de débit et/ou de la fréquence respiratoire de la respiration naturelle du patient sont utilisés au moins temporairement pour la modulation de l'assistance en pression.

7. Appareil de ventilation selon au moins l'une des revendications précédentes, **caractérisé en ce que** l'enregistrement de la courbe de pression et/ou de la courbe de débit et/ou de la fréquence respiratoire de la respiration naturelle du patient est utilisé au moins temporairement pour calculer une fréquence de sécurité pour la ventilation.

8. Appareil de ventilation selon au moins l'une des revendications précédentes, **caractérisé en ce que** la pression expiratoire positive (PEP) est réglée de 2 à 15 cm de H2O.

9. Appareil de ventilation selon au moins l'une des revendications précédentes, **caractérisé en ce que** différents profils de pression peuvent être utilisés pour l'inspiration et l'expiration.

10. Appareil de ventilation selon au moins l'une des revendications précédentes, **caractérisé en ce que** les obstructions par FOT et/ou aplatissement et/ou ronflement et/ou apnée et/ou une réponse de débit à la ventilation obligatoire sont détectées.

11. Appareil de ventilation selon au moins l'une des revendications précédentes, **caractérisé en ce que** le débit cardiaque ou la pression sanguine ou la fréquence cardiaque ou le Sp02 ou le CO2 sont déterminés par le biais d'un module adaptable et les grandeurs mesurées du débit cardiaque ou de la pression sanguine ou de la fréquence cardiaque ou du SpO2 ou du CO2 sont utilisées pour le calcul de l'assistance en pression.

12. Appareil de ventilation selon au moins l'une des revendications précédentes, **caractérisé en ce que** la régulation de la puissance d'un humidificateur relié à l'appareil de ventilation est effectuée en fonction de la mesure de l'assistance en pression.

13. Appareil de ventilation selon au moins l'une des revendications précédentes, **caractérisé en ce que** dans une phase d'accoutumance initiale, une assistance en pression plus faible que celle calculée comme optimale est appliquée.
